# EUROPEAN PATENT APPLICATION

(11) **EP 2 815 806 A1**
(43) Date of publication of application: **24.12.2014**
(21) Application number: 13172242.3
(22) Date of filing: 17.06.2013
(51) Int. Cl.: B01D 61/44, B01D 61/50, B01D 61/52, B01D 61/58, C12M 1/00, C12P 7/56, B01D 61/14

(54) **Apparatus and method for product recovery from fouling feeds by electrodialysis**

(71) Applicant: VITO NV (Vlaamse Instelling voor Technologisch Onderzoek NV), 2400 Mol (BE)
(72) Inventor: Brauns, Etienne, 2400 Mol (BE); de Wever, Heleen, 2260 Westerlo (BE)
(74) Representative: Pronovem

(57) **Abstract**

Apparatus (10) for recovering compounds from a feed by electrodialysis, comprising an anode (1), a cathode (2) and a plurality of ion exchange membranes (3, 4) arranged between the anode and the cathode so as to form a stack of successive electrodialysis cells (17). Each cell comprises a feed compartment (5), a concentrate compartment (6) and at least two ion exchange membranes (3, 4), being a cation exchange membrane (3) and an anion exchange membrane (4), or a bipolar membrane (9) and either one of a cation exchange membrane (3) and an anion exchange membrane (4). The feed compartment (5) and the concentrate compartment (6) of each cell are separated by one of the at least two ion exchange membranes. According to the invention, at least one porous filtration membrane (11, 12) is stacked in the feed compartment (5) of each cell to form a barrier between the feed stream and one of the ion exchange membranes. A method for recovering compounds from a feed by electrodialysis is provided as well.

## Description

The present invention is related to an apparatus and related method for product recovery by electrodialysis, such as the recovery of specific compounds, in particular organic salts, from a fermentation broth.

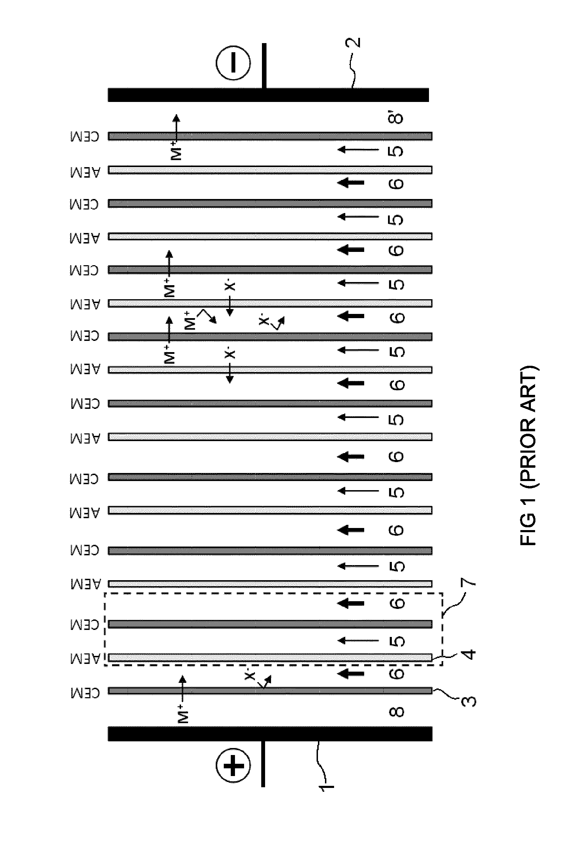

Electrodialysis (ED) is a process that is used on an industrial scale to separate salt ions from a feed (dilute) solution and move them under the influence of an applied electric potential difference through ion-exchange membranes to a collecting (concentrate) solution. The principle is illustrated in Figure 1. An electric field is created in the space between two electrodes: an anode 1 and a cathode 2. In between the two electrodes, a stack of an alternating series of cation exchange membranes (CEM) 3 and anion exchange membranes (AEM) 4 is arranged. The ion exchange membranes (CEM 3 and AEM 4) are spaced apart in the stack to form flow channels or compartments 5, 6 in between them, alternatingly for the diluate solution (compartment 5) and the concentrate solution (compartment 6). The combination of two consecutive flow compartments 5, 6 flanked by a set of one AEM and one CEM is called a cell pair 7, as indicated in Figure 1. There can be a large number of cell pairs 7 in the stacked alternating series (hundreds or even thousands). In Figure 1 an arbitrary (small) number of only 8 cell pairs is shown for illustrative purposes. The total set-up is called an ED stack.

There are hence two types of compartments per cell pair: dilute (or feed) compartments 5 through which the feed solution flows and concentrate compartments 6 through which the concentrate solution flows. In the example in Figure 1, the concentrate solution is flowing in a co-current mode with regard to the dilute solution. Other configurations are however possible.

Consecutive compartments are hence separated by an ion-exchange membrane, being a cation or an anion exchange membrane. Such membranes are typically polymeric thin films containing interstices with electrically charged functional sites made for accepting ionic species of specific charge and type, while rejecting others. Under the influence of an electric field and a concentration difference of the ionic species across the membrane, the ionic species will hop through the interstices in the membrane lattice and generate a stream of the ionic species from one side of the membrane to the other side. Example ion exchange membranes are manufactured by Astom Corporation, Japan and FuMA-Tech GmbH, Germany.

In order to guarantee an ionic conductivity in the compartments 5 and 6, an electrode electrolyte is made to (re)circulate in the outer compartments 8 and 8' between the stack and the electrodes. As a result of the presence of an electrical field between the electrodes and the presence of the conductive electrolyte, cations M⁺ can electro-migrate from the dilute compartment 5 to the concentrate compartment 6, in the direction of the negative cathode, through the cation exchange membranes 3. In an analogous way anions X- can electro-migrate from the dilute compartment 5 to the concentrate compartment 6, in the direction of the positive anode 1, through the anion exchange membranes 4. During conventional ED, the ion concentration in the concentrate stream increases while the concentration in the dilute stream decreases.

Electrodialysis is being used in the dairy and other food industry, such as for demineralization of aqueous streams, but also in the pharmaceutical and fine chemical industry. EP 0393818 describes a process for producing and purifying lactic acid involving electrodialysis. Lactate is produced in a fermentation process. The fermentation broth is then passed through a conventional electrodialysis system to obtain purified and concentrated lactate salts, which in turn are fed to a water-splitting electrodialysis system to obtain a nearly cation-free lactic acid stream. The process was implemented on an industrial scale by Eurodia Industrie SA, France. The process appears however to be more complex than described in EP 0393818. Indeed, as reported on www.eurodia.com/html/ebc.html, additional processing steps are required before the feed stream from the fermentation tank can be applied to the ED stack. In particular, the fermentation is followed by a membrane microfiltration clarification in which the bacteria are retained in the feed stream and recirculated to the fermentation tank and the permeate is fed to a divalent ion removal vessel. Any divalent ions in the permeate should be removed before applying ED in order to protect the ion exchange membranes from precipitation fouling. The ED step is a conventional ED which takes care of increasing the concentration of the lactate salt in the ED concentrate stream. The dilute stream from the conventional ED is recycled to the fermentation tank. The organic salt concentration in the conventional ED concentrate is sufficiently high to send the concentrate to a bipolar membrane electrodialysis (EDBM) stack, where the salt is converted to a base (NaOH) and the organic acid (lactic acid). The base produced by the EDBM is recycled to the fermentation tank. The organic acid is further purified by ion exchange resins and recovered.

Ion exchange membranes are susceptible for fouling resulting in a severe reduction of their capacity towards ion migration in an electrodialysis process (as in an ED stack). In Eurodia's setup described above, two distinct pre-treatment steps are therefore needed before applying ED on the raw process stream. The first step involves a pressure driven membrane filtration treatment ('clarification') in order to retain particles (including the bacteria) in the fermentation broth (retentate) and in order to produce a clean process stream (permeate), free from fouling constituents. It will be convenient to note that such a pressure driven membrane filtration requires a significant input of energy since a pressure gradient across the membrane needs to be generated. This involves thus an additional energy cost in relation to the process as a whole.

WO 2005/082495 (WO'495) describes a process for the separation of charged proteins or peptides in a composition, wherein the composition is passed between a cationic exchange membrane and an anionic exchange membrane in an ED cell. An ultrafiltration membrane is disposed between the two ion exchange membranes and the feed solution is fed on one side of the ultrafiltration membrane. On the other side of the ultrafiltration membrane, an ionic solution is made to pass which collects the charged compounds migrating from the feed solution under the influence of the applied electric field.

Whereas in WO'495 a single ED cell is used for the separation, Poulin et al. in "Improved peptide fractionation by electrodialysis with ultrafiltration membrane: Influence of ultrafiltration membrane stacking and electrical field strength", Journal of Membrane Science 299 (2007), pp. 83-90 describe a multi-cell membrane stack for the purpose. The stack is obtained by a repeating pair of one ultrafiltration membrane and one cation exchange membrane added to the cell design of WO'495. As such, a feed stream compartment and a concentrate stream compartment are provided in between each two successive ion exchange membranes. Importantly, the process is described with regard to peptide fractionation from natural streams. However, the configuration is not suitable for feeds with high fouling potential for the ion exchange membranes. Also, it will be convenient to note that in Poulin et al. the porous membrane, not the ion exchange membrane, is explicitly performing the ion separation.

Aspects of the present invention therefore envisage providing an apparatus and method suitable for product recovery from fouling feed streams, in particular feed streams obtained by fermentation processes, and which overcome the disadvantages of prior art apparatuses and methods. Aspects of the present invention envisage providing such apparatuses and methods which allow lowering the plant size, complexity and cost and/or lowering the operational cost of the plant and related process.

According to aspects of the invention, there is therefore provided an apparatus for recovering compounds from a feed by electrodialysis as set out in the appended claims.

According to other aspects of the invention, there is provided a method of recovering compounds from a feed by electrodialysis as set out in the appended claims.

Advantageous aspects of the present invention are set out in the dependent claims.

Aspects of the invention will now be described in more detail with reference to the appended drawings, wherein same reference numerals illustrate same features and wherein:

Figure 1 represents a prior art conventional electrodialysis stack;

Figure 2 represents a multi-cell ED stack according to an aspect of the present invention;

Figure 3 represents a process scheme for product recovery from a fermentation broth implementing the multi-cell ED stack of Fig. 2;

Figure 4 represents a multi-cell bipolar membrane electrodialysis (EDBM) stack according to an aspect of the invention;

Figure 5 represents a process scheme for product recovery from a fermentation broth implementing the multi-cell EDBM stack of Fig. 4.

An ion exchange membrane, as used in the present description, refers to a permselective membrane comprising electrically charged functional sites of a specific charge type, typically arranged in interstices, and which have the ability to allow particular ions diffusing through the interstices while rejecting others, at least based on their charge type (positive or negative). It will be convenient to note that, typically, an ion-exchange membrane is not fully permselective, but nevertheless can have high permselectivity (e.g. 98 % or higher). Typically, ion exchange membranes do not have an interconnected porosity, which makes ion exchange membranes not suitable for separation of compounds from a fluid based solely on particle size.

As used in the present description, an ion exchange membrane can refer to an anion exchange membrane, a cation exchange membrane, or a bipolar membrane. An anion exchange membrane refers to an ion exchange membrane having positively charged functional sites (e.g. quaternary ammonium groups) only allowing anions through it. Similarly, a cation exchange membrane refers to an ion exchange membrane having negatively charged functional sites (e.g. sulfonate groups) only allowing cations through it. It will be convenient to note that the terms anion exchange membrane and cation exchange membrane as used in the present description can refer to such exchange membranes being selective for a particular anion or cation respectively. By way of example, the term cation exchange membrane includes a proton (H⁺) exchange membrane.

A bipolar membrane as used in the present invention refers to an ion exchange membrane formed of an anion exchange layer and a cation exchange layer that are spaced apart and secured together, either physically or chemically. The anion and cation exchange layers are spaced apart by a generally thin interface which allows water to diffuse from the outside. Under the driving force of an electrical field, a bipolar membrane is able to dissociate water into hydrogen (H⁺, in fact "hydronium" H₃O⁺) and hydroxyl (OH⁻) ions.

A filtration membrane as used in the present description refers to a porous membrane having an interconnected porosity and pores of a controlled size. The pores of controlled size are (typically) present at an interface such as an outer surface of the membrane. This interface determines the selectivity, substantially based on particle size. Typically, the remainder of the membrane layer contains larger pores in order to improve permeability. Filtration membranes have the ability of separating compounds from a fluid based substantially, and possibly solely, on particle size, by allowing only those compounds through it, which fit through the pores of controlled size. A filtration membrane is generally not able to discriminate compounds based on charge type. As used in the present description, a filtration membrane can refer to a microfiltration (MF) membrane, an ultrafiltration (UF) membrane, or a nanofiltration (NF) membrane. Filtration membranes as used in the present invention can advantageously retain particles having a size larger than P, while allowing particles smaller than or equal to P through the membrane, wherein P is advantageously larger than or equal to 0.5 nm, advantageously larger than or equal to 1 nm and P is advantageously smaller than or equal to 5 µm, advantageously smaller than or equal to 1 µm. For MF membranes, P typically falls in the range between 0.05 µm and 5 µm. For UF membranes, P typically falls in the range between 5 nm and 0.5 µm. For NF membranes, P typically falls in the range between 0.5 nm and 10 nm.

It will be convenient to note that NF membranes are typically cast as a thin film on a UF type supporting membrane. In such case the physical barrier can be a combined NF/UF membrane. It will also be convenient to note that in the case of NF membranes, the discrimination is based, firstly, on the mechanical/geometrical exclusion action resulting from the pore size, and secondly, on the repulsive action of an electrical charge on the NF membrane material surface, on ions of opposite charge. However, an NF membrane is not considered to be an ion exchange membrane as used in the present description.

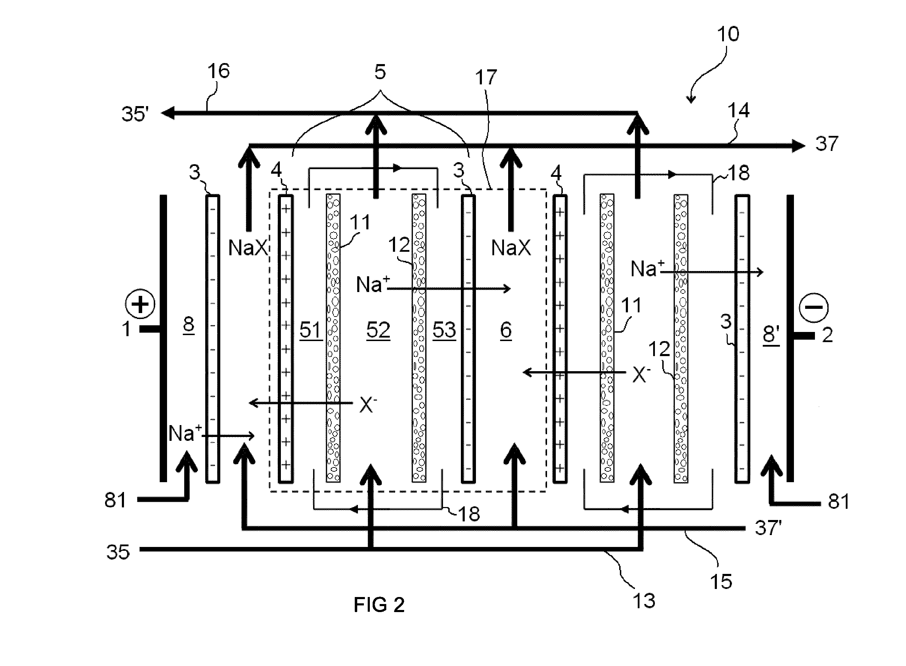

Aspects of the present invention aim at directly protecting the ion exchange membranes from fouling through the insertion of porous filtration membranes within the feed compartments of the ED stack, as shown in Fig. 2. The conventional feed compartment 5 as in Fig. 1 is thereby advantageously partitioned into three sub-compartments 51, 52 and 53 by stacking two filtration membranes 11 and 12 in the feed compartment, between the ion exchange membranes 3, 4 bordering the feed compartment 5. The feed stream is made to flow through the mid sub-compartment 52, which is delimited on both sides by the filtration membranes 11 and 12. The filtration membrane 11 between the sub-compartments 51 and 52 thus protects the AEM 4 bordering sub-compartment 51 from a direct contact with the feed stream flowing through sub-compartment 52. As a result, the filtration membrane 11 will block undesired compounds of the particle type to get into contact with the AEM. Similarly, the filtration membrane 12 between the sub-compartments 52 and 53 protects the CEM 3 bordering sub-compartment 53 from a direct contact with the feed stream flowing through sub-compartment 52. As a result, the filtration membrane 12 will block undesired compounds of the particle type to get into contact with the CEM.

It will be convenient to note that the filtration membranes 11 and 12 act here solely as a physical barrier and are thus not configured to perform a pressure driven filtration, contrary to Eurodia's clarification step. In a dead-end pressure driven filtration mode or a cross-flow pressure driven filtration mode the filtration membrane would typically show a fluid flux as a result of a significant (intended) pressure gradient across the filtration membrane. A pressure difference across the filtration membrane in pressure driven filtration is typically larger than 1 bar. Conversely, according to aspects of the present invention as illustrated in Fig. 2, any fluid flux through the filtration membranes 11 and 12 is substantially driven by the applied electric field between the electrodes 1 and 2, without there being any significant (intended) pressure gradient to provoke an additional fluid flux through the membrane. Therefore, advantageously, there is no additional hydraulic energy loss involved within the apparatuses according to aspects of the invention, compared to a conventional ED stack. In apparatuses according to the invention, a pressure difference across the filtration membranes 11 and 12 is advantageously not larger than 0.5 bar, advantageously not larger than 0.4 bar, advantageously not larger than 0.3 bar, advantageously not larger than 0.2 bar.

Hence, it is obtained that, as a result of the electrical field produced through application of a DC voltage between the anode electrode 1 and the cathode electrode 2, charged compounds will electro-migrate through the filtration membrane, from feed sub-compartment 52 to either sub-compartments 51 and 53, while other compounds will remain in the feed stream of sub-compartment 52. An important beneficial role of the porous membranes 11, 12 is thus to reject particles which are larger than the pore size of the porous membrane and to save on energy compared to a pressure driven membrane filtration situation. Therefore, the pressure driven clarification step as described above in relation to Eurodia's set-up is advantageously not needed in apparatuses according to the invention. This reduces the investment cost of the total set-up and makes the plant more compact.

Referring to Fig. 2, an apparatus 10 according to aspects of the invention comprises a stacked arrangement of ion exchange membranes forming a successive arrangement of ED cell units 17 (indicated with the dashed box in Fig. 2). In the example of Fig. 2, the ion exchange membranes are disposed such that cation exchange membranes 3 and anion exchange membranes 4 alternate in the stack, hence obtaining a disposition similar to a conventional ED stack. The stack is arranged between an anode electrode 1 and a cathode electrode 2. In operation, anode 1 and cathode 2 are connected to an electrical energy source (not shown) configured to apply a DC voltage between the anode 1 and the cathode 2, with the anode 1 being positively charged.

Each cell unit 17 comprises (at least) two ion exchange membranes 3, 4, which are spaced apart to provide for a feed compartment 5 and a concentrate compartment 6. To form the compartment 6 and sub-compartments 51-53, spacers can be used, which also care for spacing apart the ion exchange membranes 3, 4 and the porous filtration membranes 11, 12, as is known in the art. Each cell 17 advantageously comprises one feed compartment 5 and one concentrate compartment 6, which are separated by an ion exchange membrane (alternatingly a CEM 3 and an AEM 4). As the stack is formed of a succession of cells 17, a configuration is hence obtained wherein the feed compartments 5 and the concentrate compartments 6 are arranged alternatingly in the stack and are interleaved with the ion exchange membranes 3, 4, which alternate as well between CEM and AEM, such that an ion exchange membrane separates successive compartments 5, 6.

In Fig. 2, a stack of two cell units 17 is shown for illustrative purposes. The ED stack can comprise a multitude of such cells 17 disposed in parallel, typically more than fifty, advantageously more than one hundred, similar to conventional ED stacks.

As discussed above, each feed compartment 5 is divided in three sub-compartments 51-53 by disposition of two filtration membranes 11, 12 stacked in the feed compartment 5. The filtration membranes are hence stacked between an anion exchange membrane 4 and a cation exchange membrane 3 which delimit the feed compartment 5. As can be seen in Fig. 2, filtration membranes 11, 12 are only disposed in the feed compartments 5, not in the concentrate compartments 6.

Electrolyte compartments 8, 8' are provided between the anode 1 and the ED stack and between the cathode 2 and the ED stack respectively. The electrolyte compartments can be interconnected to form a circulation loop (not shown) and allow an electrode electrolyte 81, such as Na₂SO₄ to (re)circulate in (between) the electrolyte compartments 8, 8' so as to guarantee an ionic conductivity in the ED stack (electrode rinsing).

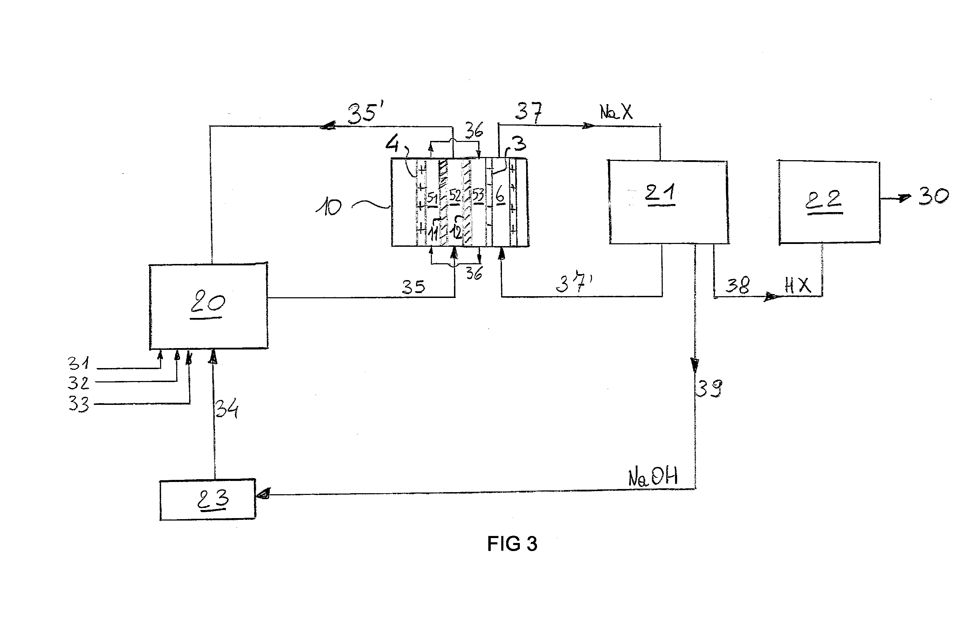

Fig. 3 illustrates a process scheme in which the apparatus 10 of Fig. 2 can advantageously be used. The scheme refers to product recovery from a fermentation broth. Sugars 31, nutrients 32 and water 33 are supplied either continuously or intermittently to a fermentation vessel (fermentor) 20. The fermentor is configured to provide the required conditions in order to produce a desired organic salt and/or organic acid by fermentation.

Fermentation is a well-known biological process in which organic constituents can be converted through microbial activity into valuable metabolic (by-)products. Typical examples are the production of corresponding organic salts and organic acids, such as lactate and lactic acid; acetate and acetic acid; succinate and succinic acid. Such biologically based technology demands the separation of the organic acids from the fermentor liquids since a too high concentration of organic acids may interfere in a negative way with the fermentation product production rate in the fermentation vessel. This negative effect of a too high organic acid concentration on the activity of the bacteria in the fermentation broth is referred to as fermentation inhibition.

There are many different organic acids which can be obtained by fermentation. A non-limiting selection of such organic acids includes both shorter and longer chain acids, mono- and dicarboxylic acids, as well as products from different types of fermentations (pure culture versus mixed culture, bacterial versus fungal, defined substrate versus organic waste streams). The following non-limiting list of organic acids can be obtained by fermentation and can be recovered by apparatuses and methods of the present invention, either as acid or as a salt: acetic acid, propionic acid, butyric acid, lactic acid, succinic acid, and itaconic acid.

The process scheme of Fig. 3 involves the possibility of adding a base 34, such as NaOH, to the fermentation broth in the fermentation vessel 20 in order to control the pH, which affects the fractional ionization of organic acids. It is known that at higher pH values, such as at a pH higher than 4, a large fraction of the organic acids are in their ionized (dissociated) state. Through the addition of the base 34 in vessel 20, the pH value can be maintained at a specific sufficiently high value in order to promote the organic acid dissociation (H⁺ X⁻) and the formation of the corresponding (dissociated) organic salt (Na⁺X⁻). The inhibiting effect of a too large concentration of organic acid is thus countered at the same time.

According to present aspects of the invention, the apparatus 10 is implemented in the process scheme of Fig. 3. The fermentation broth is drawn from fermentation vessel 20 and fed as a feed stream 35 to feed sub-compartments 52 of the ED stack of apparatus 10. As the stack comprises multiple cell units 17 arranged in parallel, the feed is distributed over the different sub-compartments 52 through an appropriate manifold represented schematically in Fig. 2 through the reference 13. A DC electric voltage difference is applied between anode and cathode of apparatus 10 to generate an electric field in the ED stack, which provides the driving force for the cations of the base 34 (Na⁺) to be transported through the filtration membranes 12 into sub-compartments 53, after which they electro-migrate through the CEMs 3 into the concentrate compartments 6 in the direction of the cathode 2. In an analogous way, the organic anions (X⁻) are transferred from feed sub-compartments 52 through the filtration membranes 11 into compartments 51, after which they electro-migrate through the AEMs 4 into concentrate compartments 6 in the direction of the anode 1.

The filtration membranes 11 and 12 have pore size characteristics such that the corresponding anion and cation that is to be recovered can pass through the membrane. In the present example, filtration membrane 11, which is disposed at the anode side of compartment 5, has pore size characteristics allowing the organic anion X- to pass through it. Filtration membrane 12, which is disposed at the cathode side of compartment 5, has pore size characteristics allowing the cations (in particular the base cations such as Na⁺) to pass through it. Importantly, the pore size characteristics of the filtration membranes 11 and 12 are selected so as to avoid that larger compounds can be transported through them, so that these larger compounds are retained in the feed stream 35. These larger compounds can be nutrients, macromolecules and bacteria which are valuable for the fermentation process. Hence, by appropriate selection of the type of filtration membrane, the need of a clarification of the fermentation broth 35 prior to the electrodialysis in apparatus 10 is avoided.

Suitable filtration membranes 11 and 12 are advantageously microfiltration, ultrafiltration and even nanofiltration membranes. Advantageously, but not necessarily, the filtration membranes 11 and 12 are of identical type and/or pore size characteristics.

In the concentrate compartments 6, which are each delimited by an AEM 4 and a CEM 3, the organic anions (X⁻) and the base cations (Na⁺) can come together and combine to an organic salt (NaX). As a result, apparatuses 10 according to the invention allow for extracting the targeted organic anion(s) from the fermentation broth while significantly increasing the concentration of the organic salt within the concentrate compartment 6.

The concentrated product stream 37 is collected from the concentrate compartments 6 through an appropriate manifold represented schematically in Fig. 2 through the reference 14. Product stream 37 can be further processed to harvest through adequate means the organic anion either as a salt or as an acid 30. By way of example, the product stream 37 can be fed to a bipolar membrane ED stack 21 in order to substitute the base cation (Na⁺) for a hydrogen ion (H⁺) and collect the organic acid HX and the base NaOH in separate streams, respectively an organic acid stream 38 and a base stream 39. In case the organic acid stream 38 needs further purification, it can be fed to a purification apparatus 22 where the stream 38 can further be purified, e.g. by passing through ion exchange resins. The base stream 39 can be fed to a base collection vessel 23, from which the base stream 34 for the fermentation vessel 20 can be drawn.

The bipolar membrane ED stack 21 can deliver a stream with a reduced NaX concentration but still assuring a sufficiently high ionic conductivity needed by the ED set-up 10, which can be recycled as feed stream 37' to the concentrate compartments 6. Stream 37' can principally comprise water. To this end, an inlet manifold 15 can be provided to distribute the stream 37' over the concentrate compartments 6. Similarly, the feed stream 35, after having passed the feed sub-compartments 52 in the apparatus 10, will comprise a lower concentration of the organic anion and the base cation, and form a dilute stream 35', which is collected through an outlet manifold 16. The dilute stream 35' is advantageously recycled to the fermentation vessel 20 in order to reduce fermentation inhibition. By so doing, the recovery of the organic anion X-is performed *in situ.* The process scheme of Fig. 3 can hence be referred to as an *in situ* product recovery concept.

Advantageously, the inlets of the sub-compartments 51 and 53 can be connected to each other, as can the outlets thereof through connection manifolds 18. Connection manifolds 18 can either connect the sub-compartments 51 and 53 within each single cell 17, or connect all sub-compartments 51 with all sub-compartments 53 within the stack. Hence, sub-compartments 51 and 53 can form a circulation loop for a liquid solution 36 - through the connection manifolds 18 - which can enhance the transport of ions from the feed sub-compartment 52 to the concentrate compartment 6.

The sub-compartments 51 and 53 together with connection manifolds 18 can form a confined or closed circulation loop with no net liquid (water) transport into or out of the closed circulation loop. In such case there is hence no net water transport between sub-compartment 52 and sub-compartments 51 and 53 and the transport of ions through the porous filtration membranes 11, 12 is hence a diffusion and/or an electro-migration process.

Alternatively, sub-compartments 51 and 53 together with connection manifolds 18 can form a non-confined or open circulation loop. In a first example of open circulation loop, a stream containing (non-fouling) nutrients (e.g. a part of stream 32 in Fig. 3) for the fermentation process in tank 20 can be injected in the open circulation loop 51-53-18 (e.g. by injecting in the liquid solution 36), rather than directly in the fermentation tank. The injection of nutrients in the circulation loop 51-53-18 can cause a net liquid transport towards the feed sub-compartment 52. The nutrients are thus allowed to be transported to the feed sub-compartment 52 through the porous filtration membranes 11, 12, which form a large area and allow for obtaining a better controlled and homogeneous dosing of such nutrients throughout the feed 35-35' and hence the fermentation broth, compared to direct injection in fermentation tank 20. In a second example of open circulation loop, a liquid (water) stream can be drained from the liquid solution 36 in connection manifolds 18. This can be useful to partially bleed a cell-free fermentation broth and as such increase cell concentration in the fermentor. In such case there will be a net liquid transport from feed sub-compartment 52 towards sub-compartments 51 and 53.

As illustrated in Fig. 2, the flow in sub-compartments 51 and 53 is countercurrent relative to each other. Other types of flow configurations, such as concurrent, are possible as well through provision of adequate manifolds 18. Also, the flow in feed sub-compartments 52 and concentrate compartments 6 is co-current relative to each other. Other types of flow configurations, such as counter-current are possible as well.

In case the feed stream 35 of fermentation broth contains an unacceptable amount of higher valence ions which need to be retained to protect the ion exchange membranes, NF membranes can be used as filtration membranes 11 and 12. It is well known that the separation activity of N F membranes is not only related to the mechanical obstruction effect by the geometrical characteristics of the pores (pore size) but also by the surface charge of the membrane polymer material. As a result, NF membranes show the capacity of also rejecting a significant amount of divalent (or higher valence) ions (e.g. Ca⁺⁺ or Mg⁺⁺) and only allowing (mainly) monovalent ions to pass through. By so doing, specific pre-treatment steps for divalent ions removal can be avoided in processes of the invention, since they can be integrated in ED apparatuses 10 according to the invention. This further reduces the installation cost and increases the compactness of the installation.

Additionally or alternatively to the use of NF membranes in rejecting an important part of divalent type cations, monovalent cation exchange membranes can be used as the cation exchange membrane 3 in apparatuses 10, in order to prevent divalent cations from being transferred to the ED stack concentrate compartments 6.

Advantageously, a proton selective membrane can be used as the CEM 3 within the set-up of Fig. 2. In such case, there would be only a transport of protons toward the concentrate compartment 6, while at the same time a steady state regarding the concentration of cations is obtained. As an advantageous result of such a set-up, the apparatus as depicted in Fig. 2 directly produces within the concentrate compartment 6 an organic acid HX concentrate stream 38 instead of the organic salt NaX stream 37. In such case, the post-treatment in a bipolar membrane ED stack 21 is not required.

It will be convenient to note that in feed compartments 5 of apparatuses 10 according to the invention, additional filtration membranes can be provided as desired in order to provide for additional sub-compartments with specific functions; i.e. the three sub-compartments 51-53 can be further segmented by stacking additional filtration membranes in the feed compartment 5.

It is possible to arrange either one, or both the filtration membranes 11 and 12 in direct contact with the corresponding ion exchange membranes (AEM 4 and CEM 3 respectively in Fig. 2). In such case, there would be no sub-compartments 51 and/or 53. It will be convenient to note that even in this case, the filtration membrane forms a barrier to avoid fouling of the corresponding ion exchange membrane.

It will be convenient to note that the processes and apparatuses according to the invention fundamentally differ from those described in WO'495 and Poulin et al. (2007) as cited above. In the present invention, electrodialysis is performed as in the conventional case, and the feed and concentrate streams are separated by an ion exchange membrane. Conversely, in WO'495 and in Poulin et al. (2007), the porous (filtration) membranes are used to perform the actual separation and a concentrate or product stream is collected in a compartment between the filtration membranes and the ion exchange membranes, which would correspond to compartments 51 and 53 in apparatuses of the present invention.

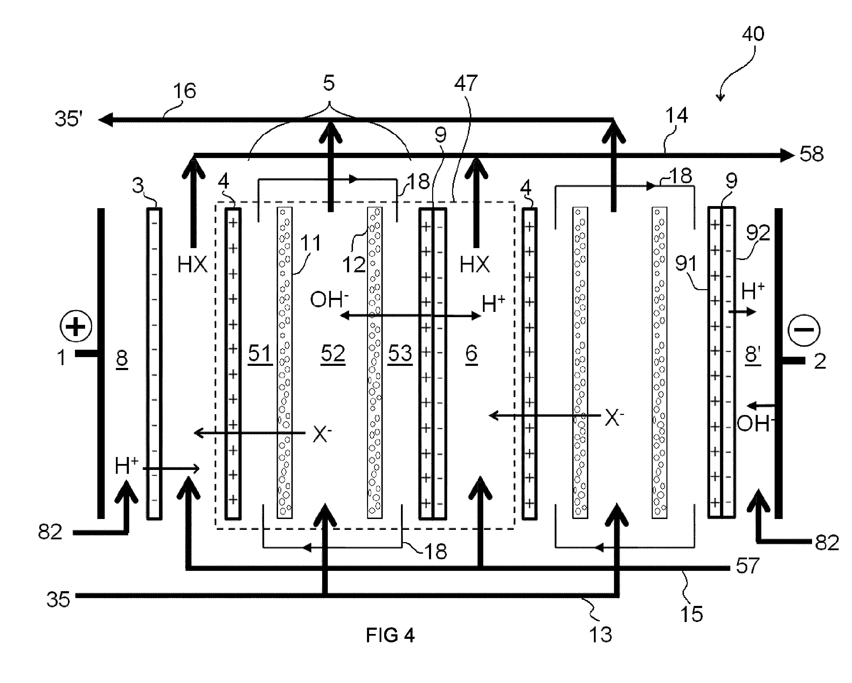

Fig. 4 illustrates an apparatus 40 according to further aspects of the invention. The addition of hydroxyl ions in the fermentation broth - through the addition of a base such as NaOH - allows, as already explained in relation to Fig. 3, for controlling the pH and degree of fractional ionization of the organic acids being produced in the fermentation broth. This however requires considerable quantities of base. Apparatus 40 enables to avoid the addition of base in the fermentation vessel through the use of bipolar membranes (BM).

It is well-known that a bipolar membrane can split H₂O into hydroxyl (OH⁻) and hydrogen (H⁺) ions in a very efficient way and at a very low energy input. The apparatus 40 is obtained by modifying apparatus 10 in that the cation exchange membranes 3 are substituted by bipolar membranes 9, all other components of the stack remaining identical, including the configuration of the streams to and from the ED stack. As a result, a bipolar membrane electrodialysis stack is obtained having successive cells 47 (indicated by the dashed box in Fig. 4) with an anion exchange membrane 4 and a bipolar membrane 9 arranged alternatingly in the stack to separate the feed compartments 5 and the concentrate compartments 6. It will be convenient to note that the bipolar membrane electrodialysis (EDBM) stack of Fig. 4 can comprise a cation exchange membrane 3 bordering the anode 1.

By orienting the BM 9 correctly with regard to the electrical field applied between anode 1 and cathode 2, the hydrogen and hydroxyl ions can be transported out of the bipolar membrane in opposite directions. In the example of Fig. 4, the BM 9 is oriented with the anion exchange side 91 facing the anode 1 and the cation exchange side 92 facing the cathode 2. In operation, under the driving force of the applied electric field, the hydroxyl ions OH-produced within the BM 9 diffuse through the anion exchange side 91 into sub-compartment 53 bordering the bipolar membrane 9 at the anode side. Filtration membrane 12 is advantageously configured to have a pore size allowing the hydroxyl ions to pass through. In such case, under the driving force of the applied electric field between anode and cathode, the hydroxyl ions are further transferred to feed sub-compartment 52. Simultaneously, the protons H⁺ produced within the BM 9 diffuse through the cation exchange side 92 into the concentrate compartment 6 bordering the BM 9 at the cathode side. At the same time, under the driving force of the same electric field, the organic anions X- migrate from feed sub-compartment 52 into sub-compartment 51 bordering the AEM 4, after which they are transported through the AEM 4 into the concentrate compartment 6, thereby combining with the hydrogen ion H⁺ into the organic acid HX.

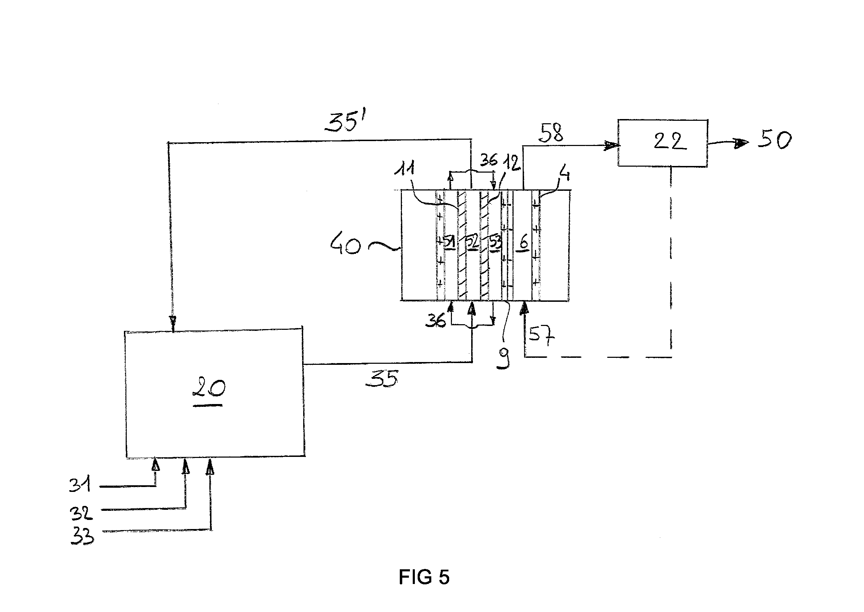

According to present aspects of the invention, the apparatus 40 is advantageously applied in a process scheme as represented in Fig. 5. This process scheme differs from the process scheme of Fig. 3 in that it does not require a separate EDBM stage 21 and actually integrates it to convert the organic salt into the acid form and to produce base for pH control through the water splitting action of the bipolar membranes. As a consequence, it also lacks the base (NaOH) circuit 37 - 39 - 34. Hence, the process scheme of Fig. 5 using the apparatus 40 can advantageously and in a compact way be applied in fermentation processes which require the addition of a base for pH control.

Hence, in operation, a fermentation broth can be drawn from fermentation vessel 20 and fed as a feed stream 35 to feed manifold 13 which distributes stream 35 over sub-compartments 52 of the EDBM stack of apparatus 40. As discussed, under the driving force of the electrical field applied between anode 1 and cathode 2, the organic anions X- electro-migrate in the direction of the positive anode 1 towards the concentrate compartments 6. The migration of the organic anions out of feed stream 35 is compensated by the diffusion of hydroxyl ions produced by the bipolar membranes 9 through the filtration membranes 12 into the feed sub-compartment 52. Having passed the feed sub-compartments 52, the feed stream, depleted of organic anions and with corrected pH due to OH- migration, is collected by the outlet manifold 16. It exits the EDBM stack as a dilute stream 35' and can advantageously be fed back to the fermentation vessel 20. In the concentrate compartments 6, the organic anions combine with the protons produced by the bipolar membranes 9 to yield the organic acid HX. To this end, a solution 57 is distributed by inlet manifold 15 over the concentrate compartments 6, which solution is arranged to collect the organic acids HX. The solution, once enriched with the organic acid, is collected as a product stream 58 at the outlet end of the concentrate compartments 6 by collecting manifold 14. As desired, product stream 58 can be fed to the purification apparatus 22 to recover the organic acid in a purified and concentrated stream 50. Possibly, the purification apparatus can provide for producing the organic acid collecting solution 57.

It will be convenient to note that the sub-compartments 51 and 53 of stack 40 can be interconnected as described in relation to Fig. 3. It is equally possible to arrange the filtration membranes 11, 12 against the respective anion exchange membrane 4 and bipolar membrane 9, as discussed in relation to Fig. 2 above.

An electrode rinsing solution 82 is circulated in electrolyte compartments 8 and 8' provided respectively between the anode 1 and the EDBM stack and between the cathode 2 and the EDBM stack so as to guarantee an ionic conductivity in the EDBM stack. The electrode rinsing solution is advantageously an aqueous solution providing the following reactions:
at the anode side: 2H₂O → 4H⁺ + O₂ + 4e⁻;
at the cathode side: 2e- + 2H₂O → H₂ + 2OH⁻.

It is thus clear that the process scheme of Fig. 5 allows for combining the conventional steps of clarification, removal of divalent ions, conventional ED and bipolar ED as illustrated by Eurodia into one single unit 40 which clearly constitutes a more advantageous and much more compact method of product recovery.

It will be convenient to note that an EDBM configuration as in apparatus 40 with the anion exchange membranes 4 substituted by cation exchange membranes 3 is also possible in an analogous way. Such a configuration can be used for recovering (organic) cationic compounds Y⁺ instead of anionic ones X⁻. In the case of harvesting a Y⁺ constituent obviously the AEM 4 in Fig. 4 needs to be substituted by a CEM 3 in order to allow for passing the Y⁺ constituent into compartment 6. This obviously also requires to have the positive anode electrode 1 at the right hand side and the negative cathode electrode 2 at the left hand side compared to the configuration of Fig. 4. In addition, it is obvious that also the orientation of the BM 9 needs to be inversed: the AEM side 91 of the BM should thus face concentrate compartment 6 while the CEM side 92 of the BM should face sub-compartment 53. By so doing, a concentrate of Y⁺OH⁻ would therefore be obtained in concentrate compartment 6.

In the special case of a non-fouling feed stream 35 with regard to the bipolar membranes 9, the filtration membrane 12 which separates sub-compartments 52 and 53 can possibly be omitted in such a way that sub-compartments 53 and 52 would constitute one single compartment (in fact sub-compartment 53 would disappear) having the feed stream 37 flowing through it (while in physical contact with the BM 9). In that case there is only one filtration membrane 11 involved protecting the anion exchange membrane (or, as the case may be, the cation exchange membrane).

It will be convenient to note that, although not illustrated in the above examples, the concentrate compartments 6 can be segmented into sub-compartments and can comprise additional ion exchange membranes and/or filtration membranes in order to provide further separation and/or concentration as is known in the art.

Even though the above examples have been illustrated with regard to streams drawn from fermentation processes, it will be clear to those skilled in the art that also feed streams containing ionic compounds to be recovered and originating from processes or reactions different from fermentation can be applied in the present invention.

## Claims

1. Apparatus (10, 40) for recovering compounds from a feed (35) by electrodialysis, comprising an anode (1), a cathode (2) and a plurality of ion exchange membranes (3, 4, 9) arranged between the anode and the cathode so as to form a stack of successive electrodialysis cells (17, 47), each cell comprising a feed compartment (5) configured for passing a stream of the feed, a concentrate compartment (6) configured for collecting the compounds recovered from the feed stream (35), and at least two ion exchange membranes (3, 4, 9), being a cation exchange membrane (3) and an anion exchange membrane (4), or a bipolar membrane (9) and either one of a cation exchange membrane (3) and an anion exchange membrane (4), wherein the feed compartment (5) and the concentrate compartment (6) of each cell are separated by one of the at least two ion exchange membranes,
**characterised in that** at least one porous filtration membrane (11, 12) is stacked in the feed compartment (5) of each cell to form a barrier between the feed stream and at least one of the at least two ion exchange membranes.

2. Apparatus (10, 40) of claim 1, wherein each cell (17, 47) comprises at least two porous filtration membranes (11, 12) being stacked in the feed compartment (5) such that the feed stream (35) is configured to pass between the at least two porous filtration membranes thereby forming barriers between the feed stream and the at least two ion exchange membranes.

3. Apparatus (10, 40) of claim 1 or 2, wherein the feed compartments (5) and the concentrate compartments (6) alternate in the stack, and wherein the ion exchange membranes (3, 4, 9) are interleaved with the feed and concentrate compartments in the stack, wherein successive interleaved ion exchange membranes alternate between a cation exchange membrane (3) and an anion exchange membrane (4), or between a bipolar membrane (9) and either one of a cation exchange membrane (3) and an anion exchange membrane (4).

4. Apparatus (10, 40) of any one preceding claim, wherein the at least one porous filtration membrane (11, 12) divides the feed compartment (5) into at least two sub-compartments, being a first sub-compartment (52) for passing the feed stream (35) and at least one second sub-compartment (51, 53) interposed between the at least one porous filtration membrane and one of the at least two ion exchange membranes, the second sub-compartment being configured for hosting an intermediate stream (36) for transporting ions, which are transported from the first sub-compartment (52) through the porous filtration membrane (11, 12), to the one ion exchange membrane (3, 4, 9).

5. Apparatus (10, 40) of claim 4, comprising a distribution manifold (13) in fluid connection with the first sub-compartments (52) and configured for distributing the feed stream (35) over the first sub-compartments, and a collection manifold (14) in fluid connection with the concentrate compartments (6) and configured for collecting the recovered compounds.

6. Apparatus (10, 40) of claim 4 or 5, wherein the second sub-compartments (51, 53) are so connected with one another to form one or more circulation loops (18) for the intermediate stream (36), such that the intermediate stream is arranged to circulate from one second sub-compartment to another.

7. Apparatus (10, 40) of any one claim 4 to 6, wherein each cell (17, 47) comprises a pair of porous filtration membranes (11, 12) being stacked in the feed compartment (5), the filtration membranes dividing the feed compartment into the first sub-compartment (52) interposed between the pair of porous filtration membranes, the first sub-compartment being configured to accept the feed stream, and a pair of second sub-compartments (51, 53) interposed between the porous filtration membranes and the ion exchange membranes, one second sub-compartment being provided at each side of the first sub-compartment.

8. Apparatus (10, 40) of claim 7, wherein the second sub-compartments (51, 53) are interposed between the first sub-compartments (52) and the concentrate compartments (6) in the stack, and wherein the second sub-compartments (51, 53) are separated from the concentrate compartments (6) by the ion exchange membranes (3, 4, 9).

9. Apparatus (10, 40) of any one preceding claim, wherein the feed compartments (5) comprise inlets and outlets for the feed stream (35), the apparatus comprising a fermentor (20) configured for hosting a fermentation process, the fermentor having an outlet configured to draw a fermentation broth from the fermentor and being in fluid connection with the inlets (13) of the feed compartments, and the outlets (16) of the feed compartments being in fluid connection with an inlet of the fermentor so as to form a circulation loop for the feed stream (35) between the fermentor (20) and the feed compartments (5).

10. Apparatus (10, 40) of any one preceding claim, wherein the porous filtration membrane (11, 12) is a microfiltration membrane, an ultrafiltration membrane, or a nanofiltration membrane.

11. Method of recovering compounds from a feed by electrodialysis, comprising:
- providing a stack of successive electrodialysis cells (17, 47) between an anode (1) and a cathode (2), each cell comprising a feed compartment (5), a concentrate compartment (6) and at least two ion exchange membranes (3, 4, 9), being a cation exchange membrane (3) and an anion exchange membrane (4), or a bipolar membrane (9) and either one of a cation exchange membrane (3) and an anion exchange membrane (4), wherein the feed compartment (5) and the concentrate compartment (6) of each cell are separated by one of the at least two ion exchange membranes,
- passing a stream (35) of the feed through the feed compartments (5),
- applying a DC voltage between the anode and the cathode,
- collecting an ionic part of the compound in the concentrate compartments (6), **characterised in that** the ionic part is transported through a porous filtration membrane (11, 12) provided in the feed compartment (5) before reaching one of the ion exchange membranes (3, 4, 9) and subsequently electro-migrates through the one ion exchange membrane to end in the concentrate compartment (6), whereas at least one second compound of different type is retained in the feed stream (35) by the porous filtration membrane.

12. Method of claim 11, wherein ions are transported through the porous filtration membrane and an intermediate stream (36) provided between the porous filtration membrane (11, 12) and the one ion exchange membrane (3, 4, 9), wherein the intermediate stream is circulated in a circulation loop (36).

13. Method of claim 11 or 12, wherein the ionic part of the compound is an organic anion.

14. Method of claim 13, wherein the organic anion is obtained by fermentation.

15. Method of any one claim 11 to 14, wherein the feed stream (35) is a fermentation broth drawn from a fermentor (20), the feed stream being recirculated to the fermentor after having passed the feed compartments (5) of the electrodialysis cells.
